# EUROPEAN PATENT APPLICATION

(11) **EP 0 850 628 A1**
(43) Date of publication of application: **01.07.1998**
(21) Application number: 97113802.9
(22) Date of filing: 09.08.1997
(51) Int. Cl.: A61F 13/15, C09J 153/02, A61F 13/56

(54) **Anatomically shaped sanitary napkin for secure topical adhesive attachment to the skin**

(30) Priority: 23.12.1996 EP 96120738
(71) Applicant: THE PROCTER & GAMBLE COMPANY, Cincinnati, Ohio 45202 (US)
(72) Inventor: Wierlacher, Stefan Alois, 60528 Frankfurt a.M. (DE)
(74) Representative: Hirsch, Uwe Thomas

(57) **Abstract**

A tridimensional disposable absorbent article having a body facing surface and a garment facing surface, a longitudinal symmetry plane, a front end edge and a rear end edge, and comprising a liquid pervious topsheet, a backsheet joined to said topsheet and an absorbent core intermediate the backsheet and the topsheet. The absorbent core has a front portion, a central portion and a rear portion, and comprises a longitudinally oriented ridge in the central and rear portion having a profile that provides for an increased body fit. The article is intended for direct attachment to the skin of the wearer and comprise an adhesive on the body facing surface.

## Description

### FIELD OF THE INVENTION

The present invention relates to disposable absorbent articles. Disposable absorbent articles are considered to be absorbent devices designed to be worn externally of the body by a user and to receive fluids discharged from the body. In particular the present invention relates to disposable absorbent sanitary napkins, catamenials, incontinence inserts, and pantiliners that are capable of providing enhanced fit for the body and reduced leakage by matching the non-planar surfaces and the non-linear grooves of the body. The disposable absorbent articles comprise an absorbent core having a front portion, a central portion and a rear portion, and comprise a longitudinally oriented ridge in the central and rear portions having a slope that decreases rearwardly. The disposable absorbent articles are intended for direct attachment to the skin of the wearer and comprise an adhesive on the body facing surface.

### BACKGROUND OF THE INVENTION

In their basic form, disposable absorbent articles comprise an absorbent core interposed between a pervious body-contacting element (alternatively referred to as a topsheet or an overwrap) and an impervious protective barrier (alternatively referred to as a backsheet). The absorbent element is, of course, intended to receive and contain the fluids discharged from the body. The body-contacting element is intended to provide comfortable and dry-feeling contact with body surfaces while allowing free passage of fluids therethrough into the absorbent element. The protective barrier is intended to prevent the fluids which are expelled or which escape from the absorbent element from soiling the user's garments.

Major disadvantages of known disposable absorbent articles intended to be worn externally of the body, e.g. leakage, wet/dirty feeling, discomfort, are related to the poor body fit achieved by these articles that are either substantially flat prior to use and must then be squeezed or folded into the right shape to follow the body surface, or, alternatively, are shaped before use, but still need improvement in order to get a better fit with the complex shapes of the user's anatomy. Moreover, most known disposable absorbent articles are intended to be applied to the panty, and typically fixed to it by an adhesive, before wearing the panty with the applied absorbent article, and this does not facilitate a good fit with the body anatomy, also owing to differences in wearing habits and in panty styles.

With respect to disposable sanitary napkins several attempts have been made in the art to improve body contact with the wearer, and hence to absorb fluids upon discharge and thereby minimize soiling, by means of a sanitary napkin having an anatomically shaped configuration with the capability of conforming to the body anatomy.

On female users this type of sanitary napkins attempts to contact and absorb menses immediately as it leaves the vestibule.

Body conforming sanitary napkins are known in the art, both those that are flat prior to use, and that are intended to shape or mold in use to match the wearer's anatomy, and those that are shaped prior to use. In European Patent Application EP 97110735.4 filed on 1 July 1997 disposable absorbent articles, particularly sanitary napkins, have been described, which are provided before use with an improved tridimensional structure capable of conforming to the various complex body shapes of the female anatomy comprising non-linear grooves and non-planar surfaces, in order to provide increased body fit and comfort, and reduced leakage.

Such disposable absorbent articles comprise a longitudinally oriented ridge in the central and rear portions having a slope that decreases rearwardly, and are preferably intended for direct application to the user's body.

Tridimensional articles according to EP 97110735.4 are capable of conforming so closely to the user's anatomy, that they are very comfortable for the user and provide a good fit in use provided they stay in place during the wearing time. To this end they can be improved as far as staying in place in use is concerned.

Due to their particular structure these articles are in fact capable of achieving a much closer contact with the wearer's body, as compared to both known flat and tridimensionally shaped products, and since they are preferably intended for direct application to the user's body, they can be improved by incorporation on their body facing surface of an adhesive for topical adhesive attachment to the wearer's body in the area where absorption of bodily fluids is desired.

The use of traditional means for fastening the article to the undergarment, though possible in this type of tridimensional articles, is in fact not particularly supporting the particular close fitting product benefits during use, since even the small relative movements between the undergarment and the body that are inevitable during the wearing time will affect the product and tend to move it, at least to a certain extent, from its preferred position in close contact with the anatomy. This effect moreover will also be influenced by the panty style and the wearing habits of the user.

The incorporation of a body adhesive on the body facing surface of a tridimensional article according to EP 97110735.4 is therefore particularly advantageous since it provides an article having an improved ability to fit and closely conform to the anatomy, with a firm and stable contact with the user's body.

Moreover, the better fit and conformability of these tridimensional articles are per se capable of improving the effectiveness of any known body adhesive incorporated in the article itself, since upon direct application to the body these articles can much more effectively fit in the right position as compared to known tridimensional articles, therefore minimizing the possibility that the article adheres to the body in a wrong position.

At the same time, the very close body fitting that can be achieved by this type of tridimensional articles makes the articles fit the same way each time, substantially with any user, and therefore the location of an acquisition zone on the article is much more predictable as compared to known products, where an acquisition point cannot be precisely defined. This allows the placement of a body adhesive on a larger portion of the body facing surface of the article, or in any case on most suitable locations in order to make the article stay in place in use, therefore achieving a better contact with the user's body without interference with the absorption and acquisition properties of the article itself.

Body adhesive compositions as those described in European Patent Application EP 97110727.1 can advantageously be used in combination with the tridimensional disposable absorbent articles, as they provide painless removal of the absorbent articles, with no residual adhesive left on the wearers body. Even more preferably the advantages for body adhesives described in European Patent Application EP 97110730.5 can be used in the context of the present invention.

It is therefore an object of the present invention to provide tridimensional disposable absorbent articles, particularly sanitary napkins, that have a structure capable of conforming to the various complex body shapes of the female anatomy comprising non-linear grooves and non-planar surfaces, which are intended for direct application to the user's body, and are attached to the user's skin by means of a body adhesive composition applied to the body facing surface of the article, preferably without causing any discomfort to the wearer upon application and particularly upon removal of the absorbent article.

It is another object of the present invention to provide a tridimensional disposable absorbent article that can be adhered more easily in the right position on the wearer's body.

It is a further object of the present invention to provide tridimensional disposable absorbent articles that are worn in such close proximity to the liquid emanating area of the wearer that liquid losses to the outside of the absorbent area are minimised or eliminated. For disposable absorbent articles worn in the crotch region of a wearer this will translate into an improved security against soiling of the surrounding skin tissue and clothing.

It is still a further object of the present invention to provide a tridimensional disposable absorbent article that is attached to the wearer's skin by means of a body adhesive providing painless removal of the absorbent article and no residual adhesive on the wearer's body upon removal.

### SUMMARY OF THE INVENTION

The present invention refers to a tridimensional disposable absorbent article having a body facing surface and a garment facing surface, a longitudinal symmetry plane, a front end edge and a rear end edge, which comprises a liquid pervious topsheet; a backsheet joined to the topsheet and an absorbent core intermediate the topsheet and the backsheet, the absorbent core comprising a front portion, a central portion and a rear portion. The body facing surface defines a line formed by the intersection of the body facing surface with the symmetry plane, the line being present in a Cartesian x-y system lying within the symmetry plane, with the x-axis defined by the two points of intersection of the longitudinal symmetry plane with the front end edge and the rear end edge, and with the body facing surface facing towards positive y values, the line having a first derivative with respect to said Cartesian x-y system. The article is such that at least one value of the first derivative of the line in the central portion of the absorbent core is larger than at least one value of the first derivative of the line in the rear portion of the absorbent core. The article further comprises on at least part of the body facing surface an adhesive for topical adhesive attachment to a wearer of the article.

### BRIEF DESCRIPTION OF THE DRAWINGS

While the specification concludes with claims particularly pointing out and distinctly claiming the present invention, it is believed that the present invention will be better understood from the following description in conjunction with the following drawings:
FIG. 1 is a perspective view of one embodiment of a sanitary napkin according to the present invention;
FIG. 2 is a cross-sectional view of the sanitary napkin of FIG. 1 on line 2-2;
FIG. 3 is a top view of the sanitary napkin of FIG. 1;
FIG. 4 is a curve taken from an anatomical section of the body of a wearer, which schematically represents the central non linear groove of the female anatomy as seen in lateral direction;
FIGS. 5a, 5b, and 5c are cross-sectional views of the sanitary napkin of FIG. 1 on lines 5a-5a, 5b-5b, and 5c-5c, respectively;
FIG. 6 is a perspective view of an alternative embodiment of a sanitary napkin according to the present invention, seen from the side that lies remote from the wearer in use.

### DETAILED DESCRIPTION OF THE INVENTION

This invention relates to tridimensional disposable absorbent articles which exhibit absorbency for bodily fluids, the protection of the user's garments from soiling, and improved physical comfort to the user, which are also easy to produce and to package and which exhibit enhanced fit to the body and better conformability to the wearer's anatomy by being provided with a tridimensional structure capable of matching the non-linear grooves and the non-planar surfaces of the female body. The articles are applied directly to the skin of a user. The disposable absorbent articles are described below by reference to a sanitary napkin or catamenial. The term "sanitary napkin", as used herein, refers to an article which is worn by females externally of the body and adjacent to the pudendal region and which is intended to absorb and contain the various body fluids which are discharged from the body (e.g., vaginal discharges, menses, and/or urine) and which is intended to be discarded after a single use. It should be understood, however, that the present invention is also applicable to other feminine hygiene or catamenial pads such as pantiliners, or other absorbent articles such as incontinence pads, and the like.

The term "use", as used herein, refers to the period of time that starts when the absorbent article is actually put in contact with the anatomy of the user.

The terms "joined" or "affixed", as used herein, encompasses configurations whereby a first member is directly connected to a second member and configurations whereby a first member is indirectly connected to a second member by connecting the first member to intermediate members which in turn are connected to the second member.

As used herein, the term "pudendal" refers to the externally visible female genitalia and is limited to the labia majora, the labia minora, the clitoris, and the vestibule.

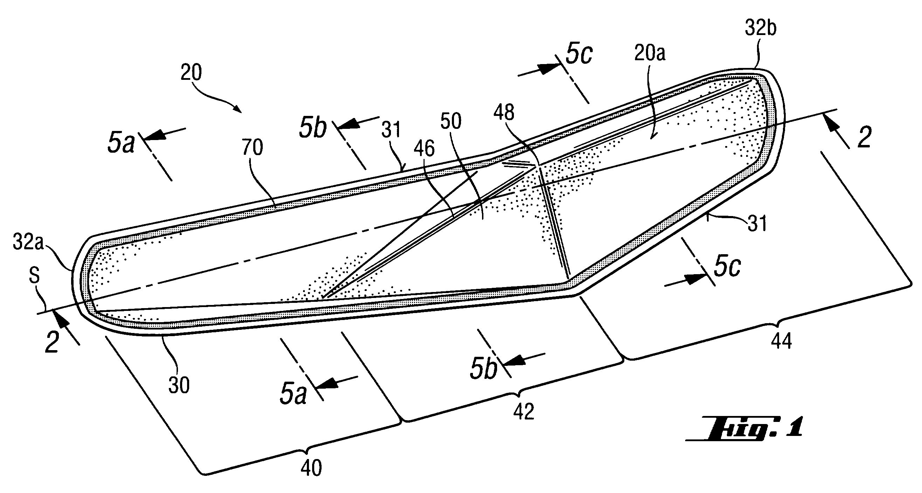

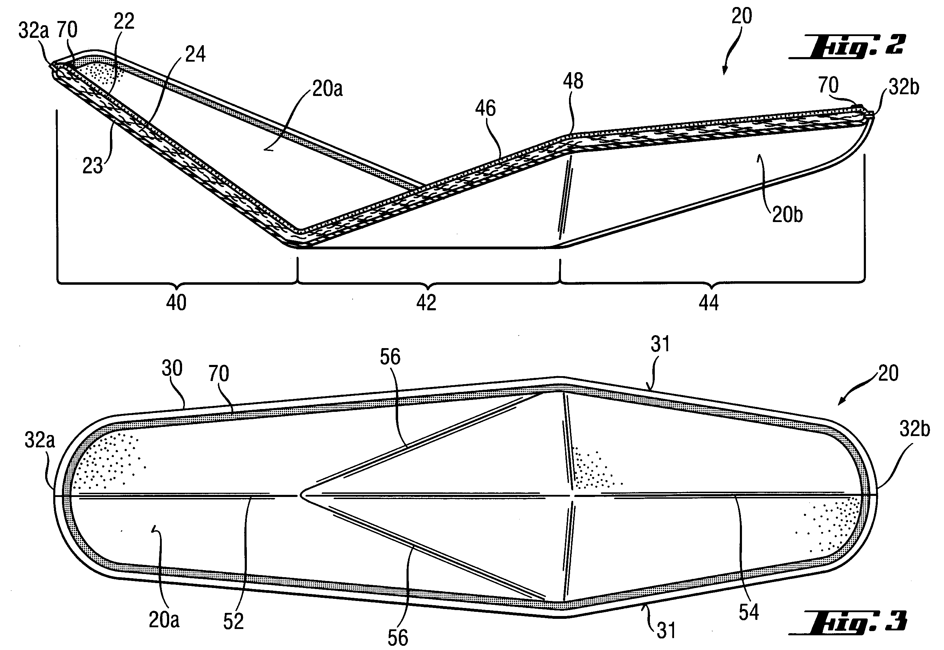

FIG. 1 is a perspective view of a sanitary napkin 20 of the present invention with its tridimensional structure before use, with most of the portion of the sanitary napkin 20 that faces or contacts the wearer, oriented towards the viewer. By saying "before use", it is meant that the sanitary napkin 20 of the present invention is provided with a tridimensional structure before it is actually worn. The sanitary napkin can nevertheless be packaged in a folded flat configuration, being subsequently unfolded to get the tridimensional shape just before wearing it. As better shown in FIG. 2, the sanitary napkin 20 comprises a liquid pervious topsheet 22, a liquid impervious backsheet 23 joined with the topsheet 22, and an absorbent core 24 positioned between the topsheet 22 and the backsheet 23.

The sanitary napkin 20 has two surfaces, a body facing or contacting surface 20a and a garment facing or contacting surface 20b. The body contacting surface 20a is intended to be worn adjacent to the body of the wearer while the garment surface 20b is on the opposite side and is intended to be directed towards the undergarment when the sanitary napkin 20 is worn, e.g. placed against it. Corresponding body facing and garment facing surfaces can also be identified in each single layer that constitutes the sanitary napkin 20, e.g., in the absorbent core 24. The sanitary napkin 20 has a longitudinal symmetry plane S. The term "longitudinal", as used herein, refers to a line, axis or direction in the sanitary napkin 20 that is generally aligned with (e.g., approximately parallel to) a vertical plane which bisects a standing wearer into left and right body halves when the sanitary napkin 20 is worn. The symmetry plane S of the sanitary napkin 20 substantially corresponds to this vertical plane that bisects the standing wearer. While it is preferred that the sanitary napkin 20 is exactly divided by the longitudinal symmetry plane S into two symmetrically equal halves, it is not excluded that the two halves be not specular. The term "transverse", as used herein, refers to a direction that is generally perpendicular to the longitudinal symmetry plane S. The term "longitudinally oriented" refers to a direction, as seen in plan view, comprised within ±45 degrees, of the longitudinal symmetry plane S; the term "transversely oriented" similarly refers to any other direction, as seen in plan view.

The terms "front" and "rear", as used herein, refer to portions or edges in the sanitary napkin 20 that are oriented towards the front and rear part of the wearer's body, respectively, when the sanitary napkin 20 is being worn.

The sanitary napkin 20 has a periphery 30, that is defined by the outer edges of the sanitary napkin 20. The longitudinal edges 31 of the sanitary napkin 20 are aligned with the longitudinal symmetry plane S, and the ends edges of the sanitary napkin 20 comprise a front end edge 32a and a rear end edge 32b. The absorbent core 24 of the sanitary napkin has a front portion 40, a central portion 42 and a rear portion 44, each one preferably corresponding to approximately one third of the total length of the absorbent core 24. Corresponding front, central and rear portions can be respectively identified in the sanitary napkin 20 also.

The sanitary napkin 20 of the present invention is tridimensional since it is provided prior to use with a tridimensional structure that is intended to match the complex body shapes of the female anatomy. The tridimensional structure has preferably a structural tridimensionality, by "structural tridimensionality" being meant that the structure cannot be completely flattened onto a flat surface while keeping its integrity, that is, without being in any case e.g. torn, crushed or squeezed. In other words, the tridimensional structure cannot be achieved by simply folding or pleating an initially flat article, but is inherently owned by the absorbent article according to the present invention. The tridimensional sanitary napkin 20 of the present invention has preferably a substantially constant thickness, that is more preferably less than 5 mm; the sanitary napkin can be therefore considered of the thin type.

While the topsheet, the backsheet, and the absorbent core may be assembled in a variety of well known configurations (including so called "tube" products or side flap products), FIG. 1 shows a preferred embodiment of the sanitary napkin 20 in which the topsheet 22 and the backsheet 23 have length and width dimensions generally larger than those of the absorbent core 24. The topsheet 22 and the backsheet 23 extend beyond the edges of the absorbent core 24 to thereby form the periphery 30 of the sanitary napkin 20.

The topsheet 22 is compliant, soft feeling, and non-irritating to the wearer's skin. Further, the topsheet 22 is liquid pervious, permitting liquid (e.g. menses and/or urine) to readily penetrate through its thickness. A suitable topsheet 22 may be manufactured from a wide range of materials such as woven and nonwoven materials; polymeric materials such as apertured formed thermoplastic films, apertured plastic films, and hydroformed thermoplastic films; porous foams; reticulated foams; reticulated thermoplastic films; and thermoplastic scrims. Suitable woven and nonwoven materials can be comprised of natural fibres (e.g., wood or cotton fibers), synthetic fibres (e.g., polymeric fibres such as polyester, polypropylene, or polyethylene fibres); or from a combination of natural and synthetic fibres.

A preferred topsheet comprises an apertured formed film. Apertured formed films are preferred for the topsheet because they are pervious to body fluids and yet non-absorbent and have a reduced tendency to allow liquids to pass back through and rewet the wearer's skin. Thus, the surface of the formed film which is in contact with the body remains dry, thereby reducing body soiling and creating a more comfortable feel for the wearer.

Suitable formed films are described in U.S. Pat. No. 3,929,135, issued to Thompson on December 30, 1975; U.S. Pat. No. 4,324,246, issued to Mullane, et al. on April 13, 1982; U.S. Pat. No. 4,342,314, issued to Radel, et al. on August 3, 1982; U.S. Pat. No. 4,463,045, issued to Ahr, et al. on July 31, 1984; and U.S. Pat. No. 5,006,394, issued to Baird on April 9, 1991. A preferred topsheet for the absorbent article of the present invention is a formed film described in one or more of the above patents and marketed on sanitary napkins by The Procter & Gamble Company of Cincinnati, Ohio as "DRI-WEAVE".

Topsheets having not a homogeneous distribution of liquid passage ways but only a portion of the topsheet comprising liquid passage ways are also contemplated by the present invention. Typically such topsheets would have the liquid passage ways oriented such that they result in a centrally permeable and peripherally impermeable topsheet for liquids.

In a preferred embodiment of the present invention, the body or exposed surface of the formed film topsheet is hydrophilic so as to help liquid transfer through the topsheet faster than if the body surface were not hydrophilic so as to diminish the likelihood that menstrual fluid will flow off the topsheet rather than flowing into and being absorbed by the absorbent core. In a preferred embodiment, surfactant is incorporated into the polymeric materials of the formed film topsheet such as is described in PCT publication WO 93/09741. Alternatively, the body surface of the topsheet can be made hydrophilic by treating it with a surfactant such as is described in U.S. 4,950,254.

Another alternative are so called hybrid topsheets which incorporate fibrous and film like structures particularly useful embodiments of such hybrid topsheets are disclosed in PCT publications WO 93/09744; WO 93/11725 or WO 93/11726.

When referring to the topsheet a multi layer structure or a mono layer structure is contemplated. The hybrid topsheet mentioned above is such a multi layer design but other multi layer topsheets such as primary and secondary topsheet designs are also considered.

The absorbent core 24 may be any absorbent means that is capable of absorbing or retaining liquids (e.g., menses and/or urine). The absorbent core 24 may be manufactured in a wide variety of sizes and shapes (e.g., rectangular, oval, hourglass, asymmetric, etc.) and from a wide variety of liquid-absorbent materials commonly used in sanitary napkins and other absorbent articles such as comminuted wood pulp that is generally referred to as airfelt. Examples of other suitable absorbent materials include creped cellulose wadding, modified cross-linked cellulose fibres (such as those described in U.S. Patent No. 5,217,445 issued to Young, et al. on June 8, 1993), capillary channel fibres (that is, fibres having intra-fibre capillary channels such as those described in U.S. Patent No. 5,200,248 issued to Thompson, et a. on April 6, 1993), absorbent foams (such as those described in U.S. Patent No. 5,260,345, issued to DesMarais, et al. on November 9, 1993 and U.S. Patent No. 5,268,244 issued to DesMarais, et al. on December 7, 1993), thermally bonded airlaid materials (such as those material described in PCT publication WO 95/10996, entitled "Catamenial Absorbent Structures Having Thermally Bonded Layers For Improved Handling of Menstrual Fluids and Their Use In Catamenial Pads Having Improved Fit and Comfort" filed in the name of Richards, et al.), absorbent sponges, synthetic staple fibres, polymeric fibres, hydrogel-forming polymer gelling agents, peat moss, tissue including tissue wraps and tissue laminates, or any equivalent materials or combinations of materials. Suitable absorbent cores comprising foams are described in European Applications 0 598 833, 0 598 823 and 0 598 834. Suitable absorbent cores comprising tissue laminates with particles of hydrogel-forming polymer gelling agents comprised therebetween are described in International Patent Applications WO 94/01069 and WO 95/17868.

The configuration and construction of the absorbent core may also be varied (e.g., the absorbent core may have varying caliper zones, e.g., profiled so as to be thicker in the centre), hydrophilic gradients, superabsorbent gradients, or lower density and lower average basis weight acquisition zones; or may comprise one or more layers or structures. The total absorbent capacity of the absorbent core should, however, be compatible with the design leading and the intended use of the sanitary napkin. Further, the size and absorbent capacity of the absorbent core may be varied to accommodate different uses such as incontinence pads, pantiliners, regular sanitary napkins, or overnight sanitary napkins. Preferably the absorbent articles of the present invention are sanitary napkins which are uniform in thickness.

The backsheet 23 and the topsheet 22 are positioned adjacent the garment facing surface 20b and the body facing surface 20a, respectively, of the absorbent core 24 and are preferably joined thereto and to each other by attachment means (not shown) such as those well known in the art. For example, the backsheet 23 and/or the topsheet 22 may be secured to the absorbent core 24 or to each other by a uniform continuous layer of adhesive, a patterned layer of adhesive, or an array of separate lines, spirals, or spots of adhesive. Adhesives which have been found to be satisfactory are manufactured by H. B. Fuller Company of St. Paul, Minnesota under the designation HL-1258 or H-2031. The attachment means will preferably comprise an open pattern network of filaments of adhesive as is disclosed in U.S. Patent 4,573,986 entitled "Disposable Waste-Containment Garment", which issued to Minetola, et al. on March 4, 1986. An exemplary attachment means of an open pattern network of filaments comprises several lines of adhesive filaments swirled into a spiral pattern such as illustrated by the apparatus and method shown in U.S. Patent 3,911,173 issued to Sprague, Jr. on October 7, 1975; U.S. Patent 4,785,996 issued to Zieker, et al. on November 22, 1978; and U.S. Patent 4,842,666 issued to Werenicz on June 27, 1989. Alternatively, the attachment means may comprise heat bonds, pressure bonds, ultrasonic bonds, dynamic mechanical bonds, or any other suitable attachment means or combinations of these attachment means as are known in the art.

The backsheet 23 is impervious to liquids (e.g., menses and/or urine) and is preferably manufactured from a thin plastic film, although other flexible liquid impervious materials can also be used. In use, the backsheet 23 is interposed between the absorbent core 24 and the user's undergarments. The function of the backsheet 23 is to prevent exudates which may be expelled from or which inadvertently bypass the absorbent core 24 from contacting and song the user's undergarments. The backsheet 23 can thus comprise a woven or nonwoven material, polymeric films such as thermoplastic films of polyethylene or polypropylene, or composite materials such as a film-coated nonwoven material. Preferably, the backsheet is a polyethylene film having a thickness of from about 0.012 mm to about 0.015 mm. Exemplary polyethylene films are manufactured by Clopay Corporation of Cincinnati, Ohio, under the designation P18-0401 and by Ethyl Corporation, Visqueen Division, of Terre Haute, Indiana, under the designation XP-39385. The backsheet 23 is preferably embossed and/or matte finished to provide a more clothlike appearance. Further, the backsheet 23 can permit vapours to escape from the absorbent core 24 (i.e., it can be breathable) while still preventing exudates from passing through the backsheet 23.

As illustrated in FIGS. 1 and 2, the sanitary napkin 20 has before use a tridimensional structure with a longitudinal oriented ridge 50 in the central and rear portions 42, 44 of the absorbent core 24, such that the line of intersection 46 of the longitudinal symmetry plane S with the body facing surface 20a has a slope decreasing rearwardly, i.e. towards the rear end edge 32b, in the central portion 42 and in the rear portion 44 of the absorbent core 24. This can be seen more clearly in FIG. 2, where the longitudinal sectional view of the sanitary napkin 20 shows the line of intersection 46 with its decreasing slope in the central and rear portions 42, 44.

The decreasing slope of said line of intersection 46 can be expressed mathematically if said line of intersection 46 is considered in a Cartesian x-y system lying in the symmetry plane S, wherein the x-axis is defined by the two points of intersection of the longitudinal symmetry plane S with the front end edge 32a and the rear end edge 32b of the sanitary napkin 20, substantially corresponding to the points indicated by numerals 32a and 32b in the cross-section view of the sanitary napkin 20 illustrated in FIG. 2, and wherein the body facing surface 20a faces towards positive y values.

With respect to this system of axes one can form the first derivative of the line of intersection 46. According to the present invention, the first derivative of this line 46 in the longitudinal direction has at least one value that is larger in the central portion 42 of the absorbent core 24 than at least one value in the rear portion 44 of the absorbent core 24. This includes the preferred case, illustrated in FIGS. 1 and 2, where the intersection line 46 is always inclined upward towards the rear end edge 32b with two different slopes in the central portion 42 and in the rear portion 44, and also alternative embodiments in which, e.g., the line of intersection 46 is inclined upward in the central portion 42 and downward in the rear portion 44.

The consecutive values of the first derivative of the line of intersection 46 can decrease continuously towards the rear end edge 32b, implying that the line of intersection 46 has a curved profile with a continuously decreasing slope, or, alternatively, the first derivative can assume different discrete values along the length of the intersection line 46. For example, it can be constant in either the central portion 42, or in the rear portion 44, or in both, the latter being the case of the embodiment illustrated in FIGS. 1 and 2, where the intersection line 46 is formed by two substantially rectilinear portions having constant slopes, with a slope change at a point 48 of the line of intersection 46 positioned where the central portion 42 of the absorbent core 24 merges the rear portion 44.

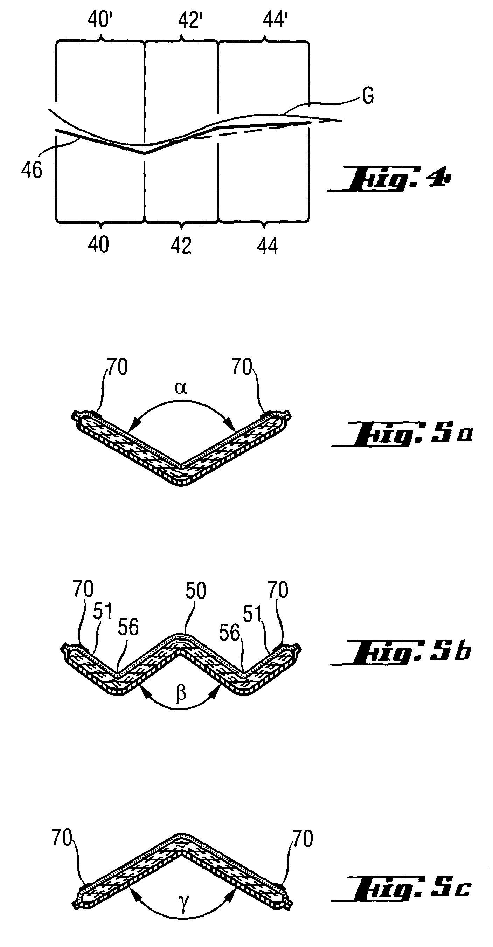

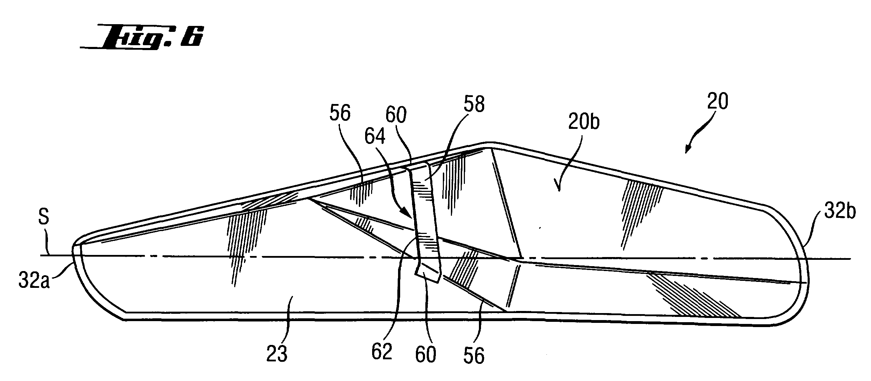

A line of intersection 46 with the above described profile provides the sanitary napkin 20 of the present invention with a longitudinally oriented ridge 50 in the central and rear portions 42, 44 of the absorbent core 24 having a longitudinal non linear profile that is intended to match in use the central non linear groove of the female anatomy extending from the labia majora to the perineum and into the gluteal groove, and having approximately the shape schematically indicated in the corresponding central and rear portions 42', 44' of the curve G, also featuring a corresponding front portion 40', illustrated in FIG. 4, where the matching profile of a line of intersection 46 in a sanitary napkin illustrated in FIGS. 1 to 3 is also shown.

The profile of the longitudinally oriented ridge 50 as defined by the line of intersection 46 with its slope decreasing rearwardly can provide the sanitary napkin 20 with an improved fit to the wearer's body. In the preferred embodiment illustrated in FIG. 1, when going from front to rear, the forward portion of the ridge 50, with a substantially constant slope, is intended to fit the groove between the labia majora. The subsequent portion of the ridge 50 that bridges the central and the rear portions 42, 44 of the absorbent core 24, with its change in slope, has a profile that is capable of matching in use the downwardly concave portion of the central non linear groove of the female anatomy in the region going from the rearward part of the labia majora to the perineum, so as to achieve a continuous contact with the body. This provides for a better comfort and for a more effective interception of the fluids as they are released from the body. Finally, the rearward portion of the longitudinally oriented ridge 50, still belonging to the rear portion 44 of the absorbent core 24 and having a constant slope in the embodiment of FIG. 1, is intended to extend between the buttocks, but owing to its slighter slope, as compared to the forward portion of the ridge, it is capable of contacting the body without causing any stress between the anatomy and this portion of the sanitary napkin, which could in turn cause discomfort, and/or prevent the desired substantially continuous contact between the ridge 50 and the wearer's anatomy along the entire length of the non linear groove extending from the labia majora up to the gluteal groove.

In other words, a ridge 50 with a profile having a slope decreasing rearwardly can get further into this non linear groove, as schematically indicated in FIG. 4. The ridge 50 with the profile indicated by the line 46 is in fact capable of following the profile of the groove, indicated by the curve G, by extending past a line, indicated with the dashed line in FIG. 4, that connects two points along the central groove of the body surface where the sanitary napkin has contact with the anatomy, e.g. the two points where the sanitary napkin contacts the body in correspondence of the forward and rearward portions of the ridge. A ridge shaped with a linear profile as those known in the art cannot extend past this line, since such a ridge substantially corresponds to this line, and hence cannot provide a continuous contact with the body along the entire length of the ridge.

In the embodiment of the present invention illustrated in FIGS. 1 and 2 the tridimensional sanitary napkin 20 preferably has a low constant thickness that is less than 5 mm, wherein the tridimensional structure is provided without the use of humps or of regions of different thickness, and it is an inherent feature of the sanitary napkin 20, rather than an added feature, achieved e.g. by bending, folding or joining together an initially planar structure.

As shown in the embodiment of the present invention illustrated in FIGS. 1 and 2, the front portion 40 of the absorbent core 24 is preferably upwardly concave, in order to better conform to the wearer's anatomy in the region of the mons pubis.

The sanitary napkin 20 illustrated in FIGS. 1 and 2 shows a particularly preferred configuration for the front, central and rear portions 40, 42, and 44 of the absorbent core 24. As viewed in transverse section the front, central and rear portions of the absorbent core 24 have respectively a V shape, a W shape, and an inverted V shape, as better shown in FIGS. 5a, 5b, and 5c, where transverse sections of the sanitary napkin 20 taken on lines 5a-5a, 5b-5b, and 5c-5c respectively of FIG. 1 are illustrated.

These different shapes provide the sanitary napkin 20 with the further capability of conforming to the wearer's anatomy in a direction substantially perpendicular to the already defined symmetry plane S. The V shape of the front portion 40 and the inverted V shape of the rear portion 44 merge together gradually in the central portion 42, where the resulting W shape is predisposed to fit the area of the labia majora and of the perineum. In use, the longitudinally oriented ridge 50 is intended to fit the longitudinal central groove as above described, while the side portions 51 bent upwardly can match the groin creases, i.e. the two grooves that are formed between the body and the legs, typically in the area where the panty elastics contact the body.

In the preferred embodiment of the present invention illustrated in FIGS. 1 and 2 the sanitary napkin 20 is provided with an increased capability of conforming to the wearer's anatomy than that simply given by the known differentiated transverse shaping of the different portions of the absorbent core 24.

The tridimensional structure of the sanitary napkin 20 prior to use is such that the width of the angle γ of the inverted V shaped portion increases towards the rear end edge 32b of the sanitary napkin 20 starting from a minimum preferred value at a position corresponding to the merging of the rear portion 44 with the central portion 42 of the absorbent core 24, where it substantially corresponds to the angle β of the central inverted V part of the W shaped central portion 42, which is in turn substantially constant along the entire length of this portion 42. Therefore the rearward portion of the ridge 50, typically positioned in use between the buttocks, can more easily widen its inverted V shape during the wearing of the product without being restrained, so providing the sanitary napkin with a better conformability to the anatomical configuration of the wearer.

A similar feature is preferably provided in the V shaped front portion 40 of the absorbent core 24, where the angle α of the V increases its width towards the front end edge 32a of the sanitary napkin 20 from a minimum preferred value at a point corresponding to the merging of the front portion 40 with the central portion 42. This will allow the portion of the sanitary napkin 20 which is closer to the front end edge 32a to more easily flatten in transverse direction during wearing in order to accommodate the relatively flat front part of the mons pubis, while still providing an overall concave shape to effectively follow the surface of the mons pubis.

The angles of the V shaped front portion 40 and/or of the inverted V shaped rear portion 44 of the absorbent core 24, and consequently of the entire sanitary napkin 20, can therefore increase towards respective end edges 32a and/or 32b up to values around 180°, in order to better accommodate the anatomy of the wearer without inducing any substantial stress in the structure, thus providing for a better fit and comfort.

The preferred feature of the angles increasing towards respective end edges in the V shaped and inverted V shaped portions is achieved by giving the front portion 40 and/or the rear portion 44 of the absorbent core 24 a cup shaped structure with any means known to the man skilled in the art. For example, in the sanitary napkin 20 of the present invention illustrated in FIGS. 1 and 2 this is achieved by cutting away a narrow V shaped portion of material centered along the longitudinal centreline of initially flat front portion 40 and rear portion 44 of the absorbent core 24, and of the topsheet 22 and the backsheet 23 as well, and having substantially the same length of the front portion 40 and of the rear portion 44, and then joining together the cut edges with known means, e.g. by thermobonding, along the junction lines identified as 52 and 54 in FIG. 3. The final tridimensional structure illustrated in FIGS. 1 and 2 is then achieved by suitably bending the non planar sanitary napkin 20, e.g. along lines of preferential bending, formed in the absorbent core 24 by means of e.g. embossments or partial cuts, such as the embossments 56 in FIG. 3, as can be readily determined by the man skilled in the art.

The presence of this preferred feature in the sanitary napkin of the present invention illustrated in FIGS. 1 and 2 can be readily ascertained when folding transversely the sanitary napkin 20 in order to superimpose the front portion 40 or the rear portion 44 of the absorbent core over the central portion 42 along a fold line that approximately in the unfolded sanitary napkin corresponds to a line separating respectively the front portion 40 or the rear portion 44 from the central portion 42: in both cases the folding line will show an angle rather than being rectilinear.

In an alternative embodiment of the present invention a tridimensional shape similar to that illustrated in FIGS. 1 to 5c can also be achieved by comprising in a disposable absorbent article a resilient insert having the desired shape, e.g. between the backsheet and the absorbent core. The insert can be comprised for example only in the central and rear portions of the absorbent article, where the ridge with the desired profile is to be provided, or can extend along the entire length of the absorbent article, in order to provide its whole shape. The resilient insert can be made of any known suitable material, e.g. absorbent or non absorbent material, and can be produced e.g. by thermoforming to get the desired tridimensional shape, preferably with a constant thickness. The insert can completely provide the tridimensional structure, or can alternatively contribute to create and to maintain said structure in an already shaped absorbent article.

According to the present invention the tridimensional absorbent article, e.g. the sanitary napkin 20, is applied directly to the skin of the user. The tridimensional structure of the absorbent article and its capability to conform and fit the anatomy of the user allow the article to stay in place during the use preferably with no need for additional means intended to fasten the article to an undergarment.

In particular, sanitary napkins are applied in the genital region of a typically female user around the area of liquid discharge. The word "skin" according to the present invention does not only relate to the specific derma of the user but include the mucous tissue as well as the hair which is typically found in the genital region of users of sanitary napkins.

In order to provide fixation of the article according to the present invention to the skin of the user a certain area on the topsheet side of the article which is facing the wearer is provided with an adhesive for topical attachment also referred to as body adhesive.

A tridimensional disposable absorbent article according to the present invention, such as the sanitary napkin 20, with its improved ability to fit and closely conform to the user's anatomy will stay in particularly close contact with the body, and will be provided by the incorporation of a body adhesive on at least part of its body facing surface with a particular stability during the use.

The direct adhesion to the wearer's skin of the tridimensional, body fitting and conforming article will hold the article in its optimal position with respect to the anatomy during the wearing time, independently of the relative movements between the body and the undergarment, since preferably no fixed connection between the article and the undergarment is provided. This also eliminates the influence of the different undergarment types and wearing habits on the body fitting of the article in use.

Since the tridimensional disposable absorbent articles according to the present invention can achieve a much closer fit with the body anatomy than known tridimensional articles, owing to their particular shape and to their capacity of conforming to the body, the combination of a body adhesive with an article of the present invention has the further advantage that the article is easily positioned, and therefore caused to adhere to the body, in the optimal position, therefore minimizing the risk of adhesion in wrong positions.

As far as the distribution of body adhesive on the body facing surface 20a of the absorbent article 20 is concerned, various designs are contemplated: for example, as illustrated in FIGS. 1 to 3, the body adhesive can be provided as a continuous strip 70 along the peripheral edge of the topsheet such that a central area of the article is left without adhesive. This will facilitate placing the article such that the liquid permeable topsheet without adhesive on it is placed adjacent the bodily liquid emanating orifice such that emanating liquid is immediately transported into the absorbent structure of the absorbent article without the possibility of leakage or spillage.

It is, however, not necessary that the body adhesive is provided in a closed circle around the periphery of the topsheet but it can be provided in incremental areas such as dots or discrete lines such that decoupling between the different places of attachment is providing additional comfort to the wearer of such articles. It can be preferred to distribute the body adhesive on portions of the body facing surface 20a of the article 20 that have low relative motion with respect to the body during the use, e.g. in the front portion 40 of the article, that corresponds in use to the wearer's mons pubis.

In general, the very close body fitting of the sanitary napkin 20 of the present invention provides for a much more predictable location of the zone of the body facing surface 20a of the article intended to be actually reached by the body fluids as they leave the body. It is therefore possible to more precisely distribute the body adhesive in a generally peripheral area of the body facing surface 20a leaving only a central area of said body facing surface 20a free of adhesive, wherein said central area is the area of the body facing surface 20a which is intended to be nearer to the body fluid emanating orifice, i.e. typically adjacent to the labia majora during the use of the article. Placement of the body adhesive on a relatively larger portion of the body facing surface 20a of the absorbent article 20 of the present invention is therefore possible, so achieving a better contact with the user's body an a better staying in place of the article during the use, without negatively interfering with the fluid absorption and acquisition capacities of the article itself.

Although any type of body adhesive known in the art can be used according to the present invention, body adhesive compositions as those described in European Patent Applications EP 97110727.1 and EP 97110730.5 are particularly preferred, since their specific physical, rheological and adhesive characteristics provide a painless removal of the absorbent article, at the same time ensuring that no residual adhesive remains on the skin or on the hairs of the wearer.

As indicated in EP 97110727.1 materials useful as body adhesives according to the present invention have rheological characteristics which are measured at a reference temperature of 37°C as body temperature and in a range of frequencies. It has been found that upon application of an article with a body adhesive the adhesive contact is formed at a low frequency, while debonding happens at the speed of removing the article. This speed is expressed as a frequency of 100 rad/s while the low frequency of forming the adhesive bond has been found to be on the order of 1 rad/s. Therefore, the frequency range for use according to the present invention is between 1 and 100 rad/s. The following set of characteristics should be satisfied:
- in the range of frequencies the percent variation of the elastic modulus G'₃₇ is lower or equal to 150 %, preferably lower than 100 % and more preferably lower than 80 %, of G'₃₇ at 1 rad/s, preferably the variation is less than 10000 Pa in absolute terms.
- in the range of frequencies the variation of the viscous modulus G''₃₇ is not greater than 10000 Pa, preferably not greater than 5000 Pa, most preferably not greater than 1000 Pa.
- the value of the ratio G'37/G''37 at least for the frequency range 1 rad/s to 100 rad/s should preferably be unity or above, more preferably 1.6 or above and most preferably 3.3 or above, while preferably not exceeding about 50.

The rheological behaviour can also be related to the values of the Glass Transition Temperature Tg. For body adhesives according to the present invention Tg should preferably be less than -15°C, more preferably less than -20°C and most preferably less than -25°C.

In order to satisfy these requirements of the above rheological and physical characteristics of a body adhesive the following formulation criteria can be used in addition. It should be noted that the most of the compositions useful as body adhesive have a substantially gel-like structure and are preferably gels. This derives from the fact that:
- the prevailing component is a material liquid at room temperature
- a macromolecular or polymeric component is present in minor quantities vs. the plasticiser. It forms, in the preferred embodiments, a three dimensional network caused by physical or chemical links between the molecules. Particularly useful physical links are the ones present in systems containing Block Thermoplastic Elastomers.

### More specifically, the compositions comprise:

- from 0.5 to 20 %, preferably 5 % to 15 %, by weight of a macromolecular polymeric substance or a mixture of such substances soluble or swellable in the below mentioned plasticiser(s). As not limiting examples such macromolecular or polymeric substances can be natural and/or synthetic such as natural gums or derivatives such as natural gums and gelatines, their derivatives and alginates; polyacrilics; polyvinyl alcohol; polyethylene oxide; polyvinylpyrrolidon (PVP) or polyvinylethers, their copolymers and derivatives; cellulose derivatives; Block Copolymer Thermoplastic Elastomers and preferably Styrenic Block Copolymers and more preferably the hydrogenated grades Styrol/Ethylene-Butylene/Styrol (SEBS), Styrene/Isoprene/Styrene (SIS), and Styrol/Ethylene-Propylene/Styrol (SEPS).
- from 51 to 99.5 % by weight of a plasticising substance or a mixture of plasticising substances, which are liquid at room temperature. As non-limiting examples the plasticiser can be water, various alcohols (like in particular glycerol), glycols, polyglycols, liquid polybutenes, natural or synthetic oils such as vegetable oils, mineral oils, or combinations thereof.
- from 0 to 600 % by weight of the macromolecular polymeric substance of a tackifying resin whose main scope is to tailor the Tg especially in systems based on synthetic polymers.
- from 0 to 10 % and more preferably form 0 to 5 % by weight of substances for facilitating and stabilising the gelation both of hydrophilic or hydrophobic liquid plasticisers. These may be for oily systems, e.g. the fatty acids of C₈ to C₂₂, their metallic salts and their polyoxo-derivatives; lanolin derivatives; silica; bentonite, montmorillonite and their derivatives; polyamides, waxes or mixtures thereof.

Common additives known in the art as preservatives, antioxidants, anti UV, pigments, mineral fillers, rheology modifiers etc. can also be comprised in quantities up to 10 % each.

When chemical crosslinks are formed in the, system, a crosslinking agent can be present preferably in quantities up to 5 % by weight. Chemical crosslinking can be formed also by mutual neutralisation of polymers having different functionalities as in the reaction between acid polyacrylics and polysaccharides.

The resulting compositions for body adhesives can be divided into three families according to the nature of the main component, i.e. the liquid plasticiser(s):
1) Hydrophobic compositions in which the plasticiser is typically an oil or blend of oils of vegetable or mineral origin and the polymer is usually a synthetic polymer, preferably an elastomer, soluble or swellable in oil(s).
2) Mixed phase compositions in which both hydrophobic and hydrophilic components, possibly in both plasticisers and polymers, form two or more separate phases. In such cases an emulsifier/surfactant is preferably present at a suitable level to form stable emulsions between the incompatible phases. For body adhesives according to the present invention it is preferably that the hydrophobic components are prevailing vs. the hydrophilic ones.
3) Hydrophilic compositions in which typically the plasticiser is water/glycerol/glycols and the like and/or mixtures thereof and the polymeric phase is of synthetic (e.g. polyacrilics) or natural (e.g. natural gums) origin or mixtures thereof.

It is to stress that, differently from what is already known in the medical field and from the cited prior art, the hydrophilic compositions are not preferred while the hydrophobic and mixed phases compositions 1) and 2) are preferred in the applications of the present invention.

This depends partially on technical reasons in the sense that many hydrophilic compositions used in the medical field show too low elastic character and cohesion for being useful in the present application. The other reason to prefer hydrophobic or mixed phase compositions is that the application of the present invention in particular in the sanitary napkin field will include a probability of contacting the body adhesive with the liquid to be absorbed. Since the liquid to be absorbed are all of a general aqueous kind contact with a hydrophilic body adhesive would result in a certain absorption of the bodily liquids into the body adhesives.

This would then have the result of changing the rheological characteristics and therefore the functionality of the body adhesive, causing a non-hygienic appearance but also would cause the bodily liquids to remain in direct skin contact over an extended period which is typically not desired by any of the disposable absorbent articles according to the present invention. In addition this may also constitute a potential drawback for the user, since some hydrophilic compositions are potentially good culture media for the growth of many micro-organisms including even pathogens.

Further, hydrophilic body adhesive also tend to be perceived as cold and wet which upon application of a fresh sanitary napkin is not in line with typical consumer expectation. Additional problems result from the fact that in particular body adhesives comprising water as the plasticiser have a tendency to dry out unless they are sealed into an impermeable package.

Tridimensional disposable absorbent articles according to the present invention can be provided with body adhesive by any of the ways usual in the art. The application of the adhesive to the body facing surface 20a of the absorbent article 20 should not cause major problems to those skilled in the art. For example, the body adhesive can be applied to the desired portion of the body facing surface 20a of the sanitary napkin 20 of the present invention by spraying it directly onto the topsheet 22.

The body adhesive on the article (as is common with panty fastening adhesives) needs to be protected prior to use. This protection can be provided by a release liner such as a siliconised or surfactant treated paper, provided this paper is a good release surface for the particularly selected body adhesive.

In principle the tridimensional disposable absorbent article according to the present invention is supported on the wearer by the body adhesive and does not require additional support to remain in place. However, it is possible to provide for example a sanitary napkin with a skid resistant coating on the backsheet side in order to prevent the sanitary napkin form gradually migrating out of position. Also even though panty fastening adhesives are not desired and hence not preferred according to the present invention they are not strictly speaking excluded in the context of the present invention.

In an alternative preferred embodiment the tridimensional disposable absorbent article of the present invention can also comprise specific means for holding and applying it directly to the body, similar to those described in European Patent Application EP 97110734.7.

FIG. 6 shows a perspective view of a sanitary napkin 20 substantially equivalent to that illustrated in FIGS. 1 to 5c, seen from the side that lies remote form the wearer in use, i.e., with the garment facing surface 20b towards the viewer. The sanitary napkin 20 of FIG. 6 further comprises means 58 for holding and applying it located on the garment facing surface 20b and being oriented transversely. The means 58 for holding and applying the sanitary napkin 20 are also referred to hereinbelow as a handling aid.

Of course the means 58 for holding and applying the sanitary napkin 20 of the present invention are also intended for use by a person taking care of a user, e.g. a nurse, who handles the sanitary napkin 20 and applies it to the user's body.

In the preferred alternative embodiment of FIG. 6 the means 58 for holding and applying the sanitary napkin 20 comprises an elongated strip of elastic film material 58 oriented perpendicularly to the longitudinal symmetry plane S and located on the garment facing surface 20b of the sanitary napkin 20, in correspondence of the central portion 42 of the absorbent core 24, at a position approximately longitudinally intermediate between the front end edge 32a and the rear end edge 32b of the sanitary napkin 20. The strip 58 is affixed to the backsheet 23 at its two spaced apart ends 60 disposed on opposite sides of the symmetry plane S, with an intermediate portion 62 being not joined to said garment facing surface 20b and defining a space 64 intended for the insertion of at least one user's finger for holding and applying the sanitary napkin 20. In the embodiment illustrated in FIG. 6, where the sanitary napkin 20 has the preferred tridimensional shape before use, the space 64 is actually comprised between the intermediate portion 62 of the strip 58 and the garment facing surface 20b of the central portion of the sanitary napkin, which is concave on its garment facing surface 20b, since it corresponds to the ridge 50 on the body facing surface 20a. Typically the spaced apart ends 60 of the strip 58 are fixed with known means, e.g., with an adhesive, or by thermobonding, to the garment facing surface 20b of the backsheet 23 at intermediate locations between each bend line corresponding to the embossments 56, and the respective longitudinal edge 31.

The user can put the sanitary napkin 20, preferably with the release liner protecting the body adhesive still in its place, on the palm of her hand with the garment facing surface 20b contacting the hand and with the front end edge 32a facing towards the wrist, at the same time inserting typically one of her fingers, e.g. the middle finger, in the space 64 between the intermediate portion 62 of the strip 58 and the backsheet 23. The user can therefore hold the sanitary napkin 20 in her open hand without exerting any force, also owing to the elasticity of the preferred material that constitutes the strip 58, with substantially the front portion of the sanitary napkin 20 lying on her palm. After exposure of the body adhesive, typically performed by removing the protective release liner, application to the body can then be easily performed by the user with a single movement of her open hand, which is simple and self-explanatory as putting the empty hand on the body.

Moreover, the movements of the hand and of the fingers allow the user to completely control the manipulation of the sanitary napkin 20 during its application to the body, making use of the tactile sensitivity of the fingers to find the right position for the sanitary napkin 20. Particularly, in the preferred embodiment of the present invention, the finger inserted in the space 64 is substantially aligned with the ridge 50 on the body facing surface 20a of the sanitary napkin 20, and therefore can provide guidance to control the proper placement of the napkin 20 on the body anatomy, i.e. with the ridge 50 suitably registered with the longitudinal non-linear groove of the female anatomy extending from the labia majora to the gluteal groove. The forward portion of the ridge can be e.g. easily identified by the user with her finger inserted in the space 64, and used as a reference to direct the sanitary napkin into an optimal position on the body.

The handling aid 58 of the preferred alternative embodiment of FIG. 6 allows to take full advantage of the body adhesive on the body facing surface 20a of the sanitary napkin 20 of the present invention, particularly when it is preferably provided on as peripheral area of the topsheet such that a central area of the article is left without adhesive. The user can in fact more easily direct the sanitary napkin 20 to the right place by means of the handling aid 58 with no need of touching the body facing surface 20a of the napkin comprising the body adhesive 70, further reducing the risk that the body adhesive may adhere to the wearer's skin in the wrong place before the sanitary napkin 20 has been properly positioned.

The handling aid constituted by the strip 58 also allows an easy removal of the hand once the sanitary napkin 20 is in place, without disturbing or modifying the position of the napkin 20.

Since in the preferred embodiment of the present invention described so far the tridimensional sanitary napkin 20 does not comprise a panty fastening system, but uses a body adhesive to hold the sanitary napkin 20 securely in contact with the user's body, the handling aid of the present invention illustrated in FIG 6 preferably also allows an easy removal and, possibly, a subsequent reapplication of the sanitary napkin 20 from the body in order to use the toilet, or to make a check of the product, or in any case in order to finally dispose of the product. The user can in fact easily grab the sanitary napkin 20 while it is being worn by positioning her hand substantially in the same way as for the application, with one of her fingers inserted in the space 64 between the not joined portion 62 of the strip 58 and the backsheet 23. The sanitary napkin 20 can therefore be taken off the body and securely held by the user; the handling aid may also be used to temporarily store the sanitary napkin, e.g. while using the toilet, on the user's hand, with no need for actually holding it with the fingers, or for exerting any force on it.

The handling aid constituted by the strip 58 allows in any event the user to handle/manipulate the sanitary napkin 20 by contacting its garment facing surface 20b only, therefore avoiding any possible contact with the body adhesive on the body facing surface 20a, and further protecting the user's hand from a possibly dirty body facing surface 20a.

In absorbent articles having a tridimensional shape before the use, such as the sanitary napkin 20 in the preferred embodiment described hereinbefore, the handling aid can also preferably contribute to keep the tridimensional shape of the article during the use, e.g. in case of body movements that can disturb the proper fit of the product, or when in general there is a risk of collapse of the body fitting tridimensional shape. Otherwise the handling aid, e.g. constituted by the strip 58 illustrated in FIG. 6, stays aligned or folded or loose on the garment facing surface 20b of the product and does not disturb the product performance.

In alternative embodiments the handling aid can be constituted by more than one strip of material, or by one or more strings, while the material can be also non elastic. The handling aid can be also constituted by a strip arranged as a loop and applied to the garment facing surface 20b of the article, or by a series of loops, intended to allow the insertion of at least one user's finger.

The handling aid can also be activated by the user, e.g. by being applied to the garment facing surface of the absorbent article just before use; alternatively, a handling aid e.g. constituted by a strip 58 can be detached e.g. at one of its ends from the garment facing surface of the absorbent article and then repositioned at a different place, in order to e.g. partially control or adapt a tridimensional shape already provided in the absorbent article, or to modify the space 64 available for the insertion of at least one user's finger. A handling aid preferably constituted by a strip 58 could therefore be resealably attached to the garment facing surface 20b of the absorbent article, at either one or both ends 62, e.g. by means of a resealable adhesive, or of a mechanical fastener of the hook and loop type, such as that marketed under the tradename VELCRO. A handling aid in form of a loop could be modified by the user in order to change the diameter of the loop, and hence the space available for the insertion of the finger(s).

The handling aid of the present invention does not necessarily extend across the entire width of the absorbent article, in order to define a suitable space for the insertion of at least one users finger, which is capable of achieving a sufficiently firm fit with said at least one finger.

As illustrated in the preferred embodiment of FIG. 6, the handling aid does not extend in longitudinal direction over a major portion of the length of the disposable absorbent article; preferably, it extends over less than 10% of said length, being more preferably a narrow strip with a width, extending in said longitudinal direction, of about 1 cm.

The absorbent article of the present invention may further comprise an odour-control material for controlling unpleasant odours associated with absorbed body fluids.

Any known odour-control agent or any combination thereof that can be suitably included in a disposable absorbent article, including other materials such as binders and/or substrates, can be comprised in the absorbent article of the present invention as the odour-control material. Active carbon coated with or in addition to other odor control agents, in particular suitable zeolite or clay materials, can be e.g. incorporated in the absorbent structure.

The odour-control material can be incorporated into the absorbent article by methods known in the art, for example layered on or into the absorbent core or mixed within the absorbent core, and in any desired form, e.g. as discrete particles.

The incorporation of odour control material in the absorbent articles of the present invention is particularly advantageous since the application of the absorbent article directly to the skin of the wearer by means of the body adhesive can provide an odour seal which prevents odours of the absorbed liquid or forming from the absorbed liquid to reach beyond the absorbent article.

The absorbent articles of the present invention, particularly the sanitary napkin 20, have a length that can range among the typical values commonly used for different sizes of said sanitary articles intended for substantially external disposition adjacent to the body of the wearer. Particularly, the central and rear portions 42 and 44 of the absorbent core 24 do not have a length which is smaller than the total maximum length of the labia majora of an average user.

Other alternative means to provide a disposable absorbent article of the preferred tridimensional structure, other than cutting, joining and folding, as already explained, may be achieved by providing an absorbent article with an extensible, elastic portion that can be deformed to yield a structure equivalent to the preferred one by e.g. stretching it in transverse direction in the central and rear portions of the absorbent core.

In an alternate embodiment of the present invention, the tridimensional absorbent article may have two flaps (not shown), each of which are adjacent to and extend laterally from the side edge of the absorbent core. The flaps are configured to drape over the edges of the wearer's panties in the crotch region so that the flaps are disposed between the edges of the wearer's panties and the wearer's thighs. The flaps help serve to prevent soiling of the wearer's body and panties by menstrual fluid, preferably by forming a double wall barrier along the edges of the panty.

The flaps may be constructed of various materials including materials used for the topsheet 22, backsheet 23, combinations thereof, and may be a laminate having tissue in the centre. Further, the flaps may be a separate element attached to the main body of the tridimensional absorbent article or can comprise extensions of the topsheet 22 and/or backsheet 23. It is recommended, however, that the flaps have a liquid impervious backsheet to prevent body fluids which reach the flaps from soiling the edges of the wearer's panties.

According to the present invention, a body adhesive can also be applied on e.g. at least a portion of the body facing surface of said flaps, in order to make the flaps adhere to the inner part of the wearer's thighs. This would allow the flaps to stay outside the panty leg elastics when the panty is pulled up, so preventing the flaps from being crumpled or folded towards the garment facing surface of the absorbent article, instead of draping over the edges of the wearer's panties.

Preferred flaps that are suitable or adaptable to the tridimensional absorbent article of the present invention are disclosed in U.S. Pat. No. 4,687,478 issued to Van Tilburg on Aug. 18, 1987; U.S. Pat. No. 4,589,876 issued to Van Tilburg on May 20, 1986; and US. Pat No. 4,608,047 issued to Mattingly on Aug. 26, 1986.

Optionally, the tridimensional absorbent article may comprise components that naturally wrap the sides of a wearer's panties. Sanitary napkins having components that naturally wrap the sides of a wearer's panties suitable for use with the tridimensional absorbent article of the present invention are disclosed in U.S. Patent No. 5,584,829 entitled "Absorbent Article having Panty Covering Components that Naturally Wrap the Sides of Panties", issued to Lavash, et al. on Dec. 17, 1996 and U.S. Patent No. 5,558,663 entitled "Absorbent Articles Having Undergarment Covering Components with Zones of Extensibility", issued to Weinberger, et al. on Sep. 24, 1996.

In another embodiment of the present invention the tridimensional absorbent article can also comprise side cuffs, e.g. as described in European Patent Application EP 96120739.6.

In further alternate embodiments of the present invention the tridimensional absorbent article can also comprise additional elements, such as an acquisition layer or a secondary topsheet positioned between the topsheet 22 and the absorbent core 24 or, alternatively, in any other suitable position.

Although the disposable absorbent article of the present invention has been described with reference to a sanitary napkin, it can be used beneficially in the context of other disposable absorbent articles such as panty liners and incontinence articles. The disposable absorbent article may thus also have all those features and parts which are typical for products in the context of their intended use.

## Claims

1. A tridimensional disposable absorbent article having a body facing surface and a garment facing surface, a longitudinal symmetry plane, a front end edge and a rear end edge, and comprising a liquid pervious topsheet, a backsheet joined to said topsheet and an absorbent core intermediate said topsheet and said backsheet, said absorbent core having a front portion, a central portion and a rear portion, said body facing surface defining a line formed by the intersection of said body facing surface with said symmetry plane, said line being present in a Cartesian x-y system lying within said symmetry plane, with the x-axis defined by the two points of intersection of said longitudinal symmetry plane with said front end edge and said rear end edge, and with said body facing surface facing towards positive y values, said line having a first derivative with respect to said Cartesian x-y system, wherein at least one value of said first derivative of said line in said central portion of said absorbent core is larger than at least one value of said first derivative of said line in said rear portion of said absorbent core,
said article being characterized in that:
it comprises on at least part of said body facing surface an adhesive for topical adhesive attachment of said article to a wearer of said article.

2. A disposable absorbent article according to claim 1, characterized in that all values of said first derivative in said central portion are larger than the values of said first derivative in said rear portion.

3. A disposable absorbent article according to claims 1 or 2, characterized in that said first derivative is constant either in said central portion or in said rear portion, or in both.

4. A disposable absorbent article according to any preceding claim, characterized in that the transverse section of said article in said front portion is V shaped defining an angle α, in said central portion is W shaped defining an angle β, and in said rear portion is inverted V shaped defining an angle γ, wherein the angle α of the V in said front portion increases towards said front end edge, and the angle γ of the inverted V in said rear portion increases towards said rear end edge.

5. A disposable absorbent article according to any preceding claim, characterized in that:
- said adhesive has an elastic modulus at a temperature of 37°C (100°F), G'₃₇, and has a viscous modulus at a temperature of 37°C (100°F), G''₃₇, wherein G'₃₇ is less than 20000 Pa, and G''₃₇ is less than 15000 Pa,
- said adhesive being selected to have a dynamic elastic behaviour such that the difference, Δ G'₃₇, of G'₃₇ at a frequency of 1 rad/sec and G'₃₇ at a frequency of 100 rad/sec is not greater than 150 % of G'₃₇ at a frequency of 1 rad/sec, preferably not greater than 10000 Pa, and
- said adhesive being selected to have a dynamic viscous behaviour such that the difference, Δ G''₃₇, of G''₃₇ at a frequency of 1 rad/sec and G''₃₇ at a frequency of 100 rad/sec is not greater than 10000 Pa, preferably not greater than 5000 Pa, and wherein the value of the ratio G'₃₇ over G''₃₇ in the frequency range 1 - 100 rad/s is greater or equal to 1, preferably greater or equal to 1.6 and most preferably greater or equal to 3.3.

6. A disposable absorbent article according to claim 5 wherein said adhesive is a composition of materials comprising
- from 51 % to 99.5 % by weight of a plasticising compound or composition which is liquid at 20°C;
- from 0.5 % to 20 % by weight of a polymeric compound or composition which is solvable or swellable in said plasticising compound or composition;
- a tackifying resin in an amount of from 0 % to 600 % by weight of said polymeric compound or composition.

7. Absorbent article according to claim 5 or 6 wherein said adhesive is at least partially hydrophobic, preferably 80 % by weight of said adhesive consist of hydrophobic components and most preferably all components of said adhesive are hydrophobic.

8. A disposable absorbent article according to any preceding claim, characterized in that said disposable absorbent article is a sanitary napkin or a pantiliner, and that said body adhesive is provided in a peripheral area of said body facing surface leaving at least a central area of said body facing surface free of adhesive, said central area being that area of said body facing surface which is adjacent to the labia majora during use of said disposable absorbent article.
